# EUROPÄISCHE PATENTSCHRIFT

(11) **EP 1 261 690 B1**
(45) Veröffentlichungstag und Bekanntmachung des Hinweises auf die Patenterteilung: **09.03.2005**
(21) Anmeldenummer: 01925300.4
(22) Anmeldetag: 09.03.2001
(51) Int. Cl.: C12M 1/32, C12M 1/28

(54) **VORRICHTUNG ZUM ZÜCHTEN VON KEIMKULTUREN**
DEVICE FOR GROWING GERM CULTURES
DISPOSITIF POUR ENTRETENIR DES CULTURES DE GERMES

(30) Priorität: 10.03.2000 DE 10011310
(43) Veröffentlichungstag der Anmeldung: 04.12.2002
(73) Patentinhaber: Micro Med Gesellschaft für Angewandte und Experimentelle Mikrobiologie mbH, 55288 Armsheim (DE)
(72) Erfinder: BEMB, Wendelin, 55288 Armsheim (DE)
(74) Vertreter: Quermann, Helmut, Dipl.-Ing.
(86) Internationale Anmeldenummer: PCT/DE2001/000879
(87) Internationale Veröffentlichungsnummer: WO 2001/066686

(56) Entgegenhaltungen:
- CH-A- 555 889
- US-A- 4 247 634
- US-A- 4 659 673

## Beschreibung

Die Erfindung betrifft eine Vorrichtung zum Züchten von Keimkulturen, mit einer Schale und einem relativ zu dieser drehbaren Deckel, die beim Züchten der Keimkulturen ein geschlossenes System bilden, wobei auf den Boden der Schale ein Nährboden für die Keime aufgebracht ist und Mittel zum Beimpfen des Nährbodens mit Keimen vorgesehen sind. Unter Keimen werden im folgenden Bakterien und Pilze verstanden.

Zum Züchten von Keimkulturen finden sogenannte Petrischalen Verwendung. Es handelt sich um flache Schalen, wobei auf den Boden der Schale ein Nährboden, insbesondere ein solcher aus Agar, für die Keime aufgebracht ist Der Nährboden wird manuell mit Keimen beimpft, indem beispielsweise eine an einem unteren Ende eines Instrumentes angebrachte Impföse, die mit Keimen versehen ist, mäanderformig über die Oberfläche des Nährbodens gezogen wird. Dieses Aufbringen der Keime erfolgt vornehmlich manuell und ist damit einerseits aufwendig, andererseits lassen sich durch das unsystematische Aufbringen Ergebnisse nur schlecht reproduzieren.

Bei Verwendung von Petrischalen wird die Schale nach dem Beimpfen des Nährbodens mittels des Deckels lose verschlossen. Es handelt sich somit nicht um ein geschlossenes System in Sinne von abgeschlossen. Ein solches wäre aber erforderlich, um eine Arbeit, die bislang in medizinischen Labors erfolgt, in Arztpraxen zu verlagern.

Das Züchten von Keimkulturen in einem geschlossenen System ist in der DE 196 31 997 A1 beschrieben. Dieses beschreibt im Sinne des Oberbegriffes des Patentanspruches 1 eine Vorrichtung zum Züchten von Keimkulturen, mit einer Schale und einem relativ zu dieser drehbaren Deckel, die beim Züchten der Keimkulturen ein geschlossenes System bilden, wobei auf den Boden der Schale ein Nährboden für die Keime aufgebracht ist und Mittel zum Beimpfen des Nährbodens mit Keinem vorgesehen sind. Die beschriebene Vorrichtung findet für den besonderen Fall der bemannten oder unbemannten Raumfahrt Verwendung. Um unter diesen Voraussetzungen Experimente durchführen zu können, ist ein geschlossenes System unabdingbar. So ist insbesondere ein normales Pipettieren nicht möglich, weil Flüssigkeitstropfen aufgrund der Schwerelosigkeit nicht zuverlässig auf den Nährboden gelangen. In der bemannten Raumfahrt verlangen die Sicherheitsanforderungen den hermetischen Einschluß aller als gefährlich eingestuften Substanzen. Bei der Vorrichtung ist deshalb vorgesehen, die Schale mit dem Deckel gasdicht zu verschließen und im Deckel einen Sprühkopf und ein Druckausgleichselement vorzusehen. Es ist somit erforderlich, zunächst die Keime im Sinne einer Prozeßflüssigkeit in den Sprühkopf einzubringen. Aufgrund der geometrischen Verhältnisse der Schale ergibt sich je nach dem Winkel und Durchmesser der Schale die Notwendigkeit, die benetzte Fläche durch mehrmaliges Sprühen zu vergrößern. Hierzu kann der Deckel durch eine Drehbewegung in unterschiedliche Positionen gebracht werden.

Das Züchten von Keimkulturen in einem geschlossenen System, bei dem eine Schale und ein auf dieser aufgesetzter, nicht drehbarer Deckel Verwendung findet, ist aus der EP 0.597.837 B1 bekannt.

Offene Systeme, die im Zusammenhang mit dem Züchten bzw. Übertragen von Keimen/Keimkulturen Verwendung finden, sind aus dem Stand der Technik hinlänglich bekannt:

In der DE-OS 29 27 141 ist eine Vorrichtung zum Übertragen von Kolonien von Mikroorganismen, insbesondere von Primärnährböden auf Selektivnährböden, beschrieben. Hierbei findet ein Stempelgerät mit einer Vielzahl von Borsten Verwendung, derart, dass sie beim Stempelvorgang mit ihren Stirn- oder Haftflächen auf dem oder den Primärnährböden und auf den Selektivnährböden im wesentlichen senkrecht von oben flächig zur Anlage kommen. Die Borsten sind an einer ebenen Tragplatte im wesentlichen parallel nebeneinander angeordnet und haben eine solche Länge, dass die Stirn- oder Haftflächen der Borsten in einer zu der Tragplatte vorzugsweise parallelen gemeinsamen Ebene liegen. - Aus der DE 32 18 857 C2 ist eine Vorrichtung zur Herstellung von Zellsuspensionen zwecks Isolierung von Mutanten bekannt. - Gemäß der DE 195 20 420 A1 erfolgt die Beimpfung von Nährböden mit Keimsuspensionen mittels Pipetten, wobei die Pipetten konzentrisch zum kreisförmigen Boden der Schale in radialem Abstand zueinander geführt werden.- Aus der DE 198 26 244 A1 ist ein Gerät zur Aufnahme und Übertragung biologischer Proben bekannt, das eine Vielzahl gesteuert beweglicher nadelförmigen Probenaufnahmeelemente aufweist.

Zum Stand der Technik wird des weiteren auf die Druckschriften DE 12 10 129 A, DE 29 27 141 A1 , DE 78 15 774 U1, WO 98/41609 A1, FR 24 01 994 A1 und JP 03-254673 A verwiesen.

Aufgabe der Erfindung ist es, eine Vorrichtung gemäß dem Oberbegriff des Patentanspruches 1 so weiterzubilden, dass sie baulich einfach gestaltet ist und unkompliziert gehandhabt werden kann, wobei erreicht werden soll, dass das Züchten von Keimkulturen von medizinischen Fachlabors in die Arztpraxen verlagert werden kann.

Gelöst wird die Aufgabe bei einer Vorrichtung gemäß dem Oberbegriff des Patentanspruches 1 dadurch, dass der Deckel mit einer Öffnung zur Aufnahme des Mittels zum Beimpfen des Nährbodens versehen ist, wobei das Mittel zum Beimpfen als Rechen, Kamm oder Bürste ausgebildet ist, das in den Deckel eingesetzt wird, wobei die Zinken des Rechens bzw. Kammes oder die Borsten der Bürste bei mit der Schale verbundenem Deckel und bei in den Deckel eingesetzter Stellung des Rechens, des Kammes bzw. der Bürste den Nährboden kontaktieren.

Vor dem Beimpfen des Nährbodens mit den Keimen wird die Schale, insbesondere Petrischale, in aller Regel mit dem relativ zu dieser drehbaren Deckel verschlossen. Der Verschluß erfolgt dicht, so dass ein geschlossenes System gebildet ist. Vorzugsweise ist die Öffnung im Deckel verschlossen, beispielsweise mittels einer Klebefolie, die streifenförmig ausgebildet ist, so dass sie nur den an die Öffnung angrenzenden Randbereich des Deckels abdeckt. Die Klebefolie wird vom Deckel abgezogen, so dass sich die offene Öffnung darstellt. Das Mittel zum Beimpfen, das als Rechen, Kamm oder Bürste ausgebildet ist, wird mit den Keimen versehen, insbesondere indem der Rechen, der Kamm oder die Bürste über das zu untersuchende Gut, beispielsweise eine Stuhlprobe, bewegt oder in eine Urinprobe getaucht wird. Im Bereich des unteren Endes des Rechens oder Kamms bzw. des unteren Endes der Borsten der Bürste haften die Keime. Der Rechen, der Kamm bzw. die Bürste wird in den Deckel eingesetzt, wobei in der eingesetzten Stellung die Zinken des Rechens oder Kamms bzw. die Borsten der Bürste den Nährboden kontaktieren. In dieser Stellung ist der Rechen, der Kamm oder die Bürste mit dem Deckel verbunden, so dass bei einer relativen Drehbewegung von Deckel und Schale die Zinken bzw. Borsten mit ihrem dem Nährboden zugewandten Ende über die Nährbodenoberfläche gezogen werden, wie es vorstehend zur Impföse geschildert worden ist.

Der dichte Verschluß, der ein geschlossenes System bewirkt, kann beispielsweise auch mittels einer weiteren Klebefolie bewerkstelligt werden, die den Übergang von Deckel und Schale verschließt, indem sie auf die Schale und den Deckel geklebt ist. In diesem Zustand wird die Vorrichtung beispielsweise an die Arztpraxen ausgeliefert, so dass diese Klebefolie als Orginalitätsverschluß erkennbar ist. Es wird zunächst die Klebefolie, die die Öffnung verschließt, abgezogen und der Rechen, Kamm oder die Bürste in den Deckel eingesetzt. Dann wird die weitere, am äußeren umlaufenden Rand angebrachte Klebefolie entfernt und der Deckel relativ zur Schale um den gewünschten Winkel gedreht, beispielsweise um einen Vollkreis, so dass der Rechen, der Kamm bzw. die Bürste über das zu untersuchende Gut bewegt wird. Ist dies erfolgt, wird die weitere Folie wieder an der ursprünglichen Stelle angebracht, so dass der dichte Verschluß zwischen Deckel und Schale hergestellt ist. In diesem geschlossenen System können sich nun die Keime entwickeln.

Nach dem Einsetzen von Rechen, Kamm bzw. Bürste in den Deckel ist dessen Öffnung verschlossen. Ebenso sind Petrischale und Deckel durch die nach der Drehbewegung wieder angebrachte weitere Klebefolie verschlossen. So liegt beim Züchten der Keimkulturen ein geschlossenes System vor. Alles infektiöse Material verbleibt im Gefäß, somit nicht nur die Keime, mit denen der Nährboden beimpft wurde, sondern auch das Mittel zum Beimpfen des Nährbodens in Art des Rechens, Kamms bzw. der Bürste. Nachdem sich die Keimkulturen entwickelt haben und somit das Untersuchungsergebnis vorliegt, kann das infektiöse Material, bei geschlossenem System, vor Ort desinfiziert werden. Dies erfolgt beispielsweise durch Erhitzen in einem Autoklaven oder aber durch Einbringen eines geeigneten Desinfektionsmittels in das geschlossene System. So ist beispielsweise der Deckel mit einer weiteren Öffnung versehen, die mittels eines Gummistopfens verschlossen ist. Der Anwender in der Praxis vor Ort kann mit einer Spritze den Gummistopfen durchstechen und das Desinfektionsmittel in die mittels des Deckels verschlossene Schale einspritzen. Nach der Desinfektion kann die Vorrichtung zum Züchten der Keimkulturen als normaler Müll entsorgt werden.

Es wird als besonderer Vorteil angesehen, dass der Rechen, der Kamm oder die Bürste bei mit dem Deckel verbundener Schale von außen in den Deckel eingesetzt wird. Es ist damit keine Handhabung des infektiösen Gutes im Bereich der Schale erforderlich. Auf besonders einfache Art und Weise kann dies bewerkstelligt werden, indem der Rechen, der Kamm bzw. die Bürste eine Grundplatte aufweist, mit der die Zinken bzw. Borsten verbunden sind, wobei die Grundplatte beim Einsetzen des Rechens, des Kamms bzw. der Bürste mit dem Deckel verbunden, insbesondere verklebt wird. Es ist nur erforderlich, dass die Abmessungen der Grundplatte geringfügig größer als diejenigen der Öffnung sind, so dass die Grundplatte den Deckel im Bereich des um die Öffnung umlaufenden Randes kontaktiert und damit das geschlossene System gewährleistet ist.

Gemäß einer vorteilhaften Ausführungsform der Erfindung ist vorgesehen, dass mit der Grundplatte auf ihrer den Zinken bzw. Borsten abgewandten Seite ein Stiel verbunden ist. Der Verbindungsbereich von Stiel und Grundplatte sollte als Sollbruchstelle ausgebildet sein. Die Einheit von Rechen, Kamm bzw. Bürste und Stiel kann somit beim Aufnehmen der Keime bzw. des zu untersuchenden Gutes am Stiel ergriffen werden, und es werden die Zinken bzw. Borsten über das Gut bzw. durch dieses geführt. Die Einheit wird weiterhin am Stiel ergriffen und der Rechen, der Kamm bzw. die Bürste in den Deckel eingesetzt und hierbei mit diesem verbunden, insbesondere verklebt. Anschließend kann der Stiel in dem Bereich der Sollbruchstelle von der Grundplatte getrennt und verworfen werden.

Es ist insbesondere daran gedacht, dass die Schale rotationssymmetrisch gestaltet ist. Durch Drehen des Deckels mit dem in diesen eingesetzten Rechen, Kamm bzw. Bürste ergibt sich ein Ein- bzw. Auftrag der Keime auf den Nährboden entlang eines Kreisbogens, dessen Bogenwinkel durch den Drehwinkel des Deckels relativ zur Schale gegeben ist. Gemäß einer Weiterbildung ist die Schale durch ein oder mehrere im Bereich des Boden angeordnete, konzentrisch zur Rotationsachse von Schale und Deckel verlaufende Stege in ringfömige Nährbodenbereiche unterteilt. Es können damit mit ein und derselben Vorrichtung mehrere Keimkulturen angelegt und dargestellt werden.

Bei Verwendung eines Mittels zum Beimpfen des Nährbodens, das als Rechen oder Kamm ausgebildet ist, sind die Zinken vorteilhaft im Bereich ihres freien Endes in einem stumpfen Winkel abgewinkelt, derart, dass das freie Ende, bezogen auf die Drehrichtung des Deckels zur Schale, mit dem den Zinken vorlaufend angeordneten Bereich des Schalenbodens einen spitzen Winkel einschließt. Mit dieser Anordnung in Art eines spitzen Winkel ist gewährleistet, dass, insbesondere bei Verwendung dünnwandiger Zinken, diese nur geringfügig in den Nährboden eindringen, somit senkrecht zur Ebene des Nährbodens ausweichen können. Die Enden der Zinken sind beispielsweise als U-förmige Halbrundung, Kugel, Halbkugel, Spitze oder in Art einer impföse gestaltet. Impfösen sind bei einer entsprechenden Kalibrierung dazu geeignet, aus einer Flüssigkeit definierte Volumina aufzunehmen und diese dann zu verteilen. Es kann sich auch um unterschiedliche definierte Volumina handeln, je nach dem verwendeten Ösendurchmesser. Der Vorteil einer derartigen Kalibrierung ist in der Möglichkeit einer genauen Keimzahlbestimmung zu sehen.

Die Verbindung von Deckel und Schale, um das geschlossene System zu gewährleisten, kann auf unterschiedliche Art und Weise erfolgen. Es können beispielsweise im Bereich des Randes von Schale und Deckel Rastmittel vorgesehen sein. Es ist auch denkbar, Schale und Deckel mittels eines zentralen Haltemittels zu verbinden.

Weitere Merkmale der Erfindung sind in den Patentansprüchen, der Beschreibung der Figuren und den Figuren selbst dargestellt, wobei bemerkt wird, dass alle Einzelmerkmale und alle Kombinationen von Einzelmerkmalen erfindungswesentlich sind.

In den Figuren ist die Erfindung anhand zweier Ausführungsformen beispielsweise dargestellt, ohne auf diese beschränkt zu sein. Es zeigen:
- Figur 1: einen Schnitt durch eine erste Ausführungsform der erfindungsgemäßen Vorrichtung,
- Figur 2: eine Draufsicht der in Figur 1 gezeigten Vorrichtung,
- Figur 3: eine Seitenansicht des bei dieser Ausführungsform Verwendung findenden Rechens mit Stiel,
- Figur 4: eine Stirnansicht des in Figur 3 gezeigten Rechens mit Stiel,
- Figur 5: eine zweite Ausführungsform der erfindungsgemäßen Vorrichtung, in einem Schnitt und
- Figur 6: die in Figur 5 gezeigte Vorrichtung in einer Draufsicht.

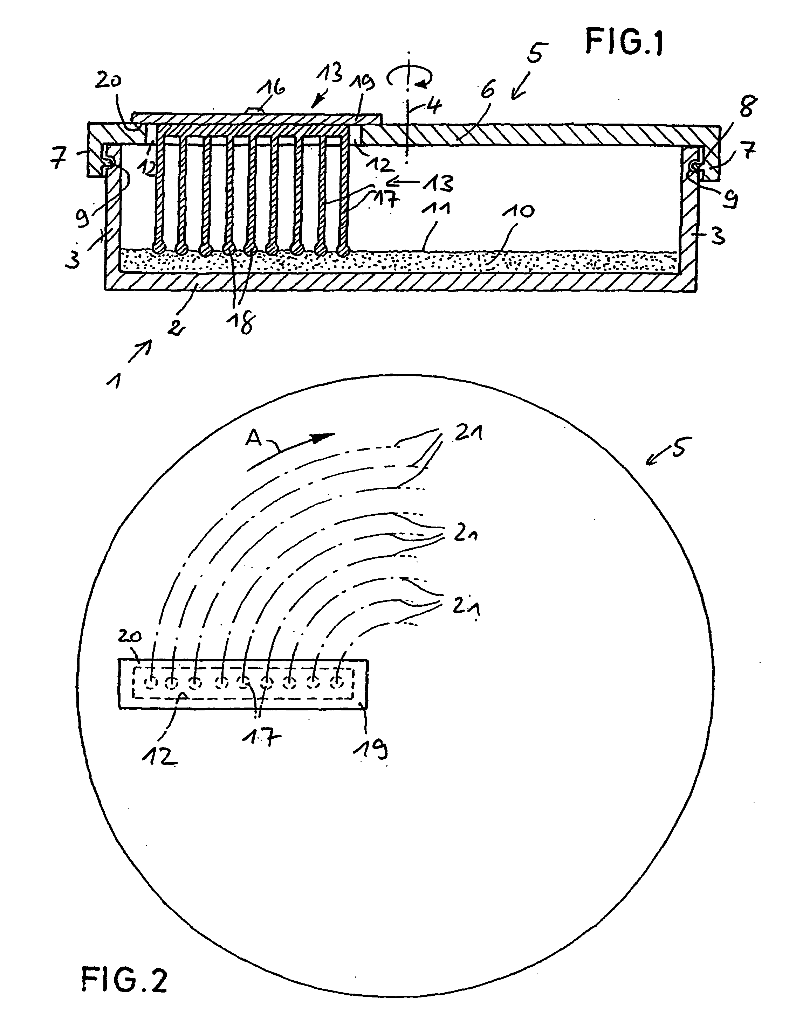

Die Vorrichtung zum Züchten von Keimkulturen gemäß der Ausführungsform nach den Figuren 1 bis 4 weist eine flache Schale 1 mit ebenem Schalenboden 2 und senkrecht hierzu angeordneter Schalenwand 3 auf. Die Schale ist bezüglich der Achse 4 rotationssymmetrisch ausgelegt, womit der Schalenboden 2 kreisförmig gestaltet ist Verschlossen wird die Schale 1 mittels eines gleichfalls bezüglich der Achse 4 rotationssymmetrisch gestalteten Deckels 5, der aus der eigentlichen kreisförmigen Deckelfläche 6 und der senkrecht zu dieser angeordneten Deckelwand 7 gebildet ist. Diese überlappt außen die Schalenwand 3 mit geringem Spiel. Innen ist die Deckelwand 7 mit einem umlaufenden Ringvorsprung 8 versehen, der abgedichtet in eine korrespondierende Ringnut 9 der Schalenwand 3 eingreift. Der Deckel 5 und die Schale 1 bilden somit ein geschlossenes System. Den Schalenboden 2 bedeckt ein Nährboden 10 für die Keime, bei dem es sich beispielsweise um Agar handelt. Dieser Nährboden 10 ist weitgehend gleichmäßig auf den Schalenboden 2 aufgebracht, so dass die dem Deckel 5 zugewandte Fläche 11 des Nährbodens 10 sowohl parallel zum Schalenboden 2 als auch parallel zur Deckelfläche 6 angeordnet ist.

Der Deckel 5 ist mit einer sich radial erstreckenden, rechteckigen Querschnitt aufweisenden Öffnung 12 versehen, deren radiale Abmessungen geringer sind als der Radius des Deckels 5. Konkret ist die Öffnung 12 im Abstand zur Achse 4 und zur Schalenwand 3 positioniert. Diese rechteckige Öffnung 12 im Deckel 5 ist mittels einer nicht gezeigten Klebefolie zugeklebt, die im Bereich der Öffnung 12 oben auf den Deckel 5 geklebt ist. Die Abmessungen der Klebefolie entsprechen etwa den rechteckigen Umrissen, die in der Figur 2 mit der Bezugsziffer 19 bezeichnet sind. - Kurz vor dem Aufbringen der Keime auf den Nährboden 10 wird die Klebefolie vom Deckel 5 abgezogen, so dass das Innere der Schale durch die Öffnung 12 zugänglich ist.

Das Aufbringen der Keime auf den Nährboden 10 erfolgt im Ausführungsbeispiel mittels eines Rechens 13. Dieser bildet mit einem Stiel 14 eine Baueinheit 15. Diese ist zwischen dem Rechen 13 und dem Stiel 14 mit einer Sollbruchstelle 16 versehen. - Zum Entnehmen der Keime von einer Probe wird die Einheit 15 am Stiel 14 erfaßt und der Rechen 13 mit seinen Zinken 17, konkret seinen freien, unteren Verdickungen 18, über bzw. durch das zu untersuchende Gut bewegt, bei dem es sich beispielsweise um menschlichen Stuhl oder menschlichen Urin handelt. Die Keime der Probe haften somit an den Zinken 17 insbesondere in dem Bereich der Verdickungen 18. Der Rechen 13 ist mit einer Grundplatte 19 versehen, die rechteckigen Querschnitt aufweist, mit Abmessungen entsprechend der Darstellung der Figur 2, die in etwa den Abmessungen der zuvor vom Deckel 5 abgezogenen Klebefolie entsprechen. In diesem Bereich ist der Deckel 5 mit einer Klebeschicht 20 versehen. Der Rechen 13 wird mit dem an den Verdickungen 18 haftenden, zu untersuchenden Gut, das die Keime aufweist, in die Öffnung 12 des Deckels 5 eingesetzt, wobei die Grundplatte 19 in der in den Figuren 1 und 2 ersichtlichen Position mit dem Deckel 5 verklebt und somit fixiert ist. Aufgrund der die Öffnung 12 überlappenden Grundplatte 19 ist der Deckel 5 in diesem Bereich dicht verschlossen. Dann wird der Stiel 14 der Baueinheit 15 im Bereich der Sollbruchstelle 16 abgebrochen und verworfen.

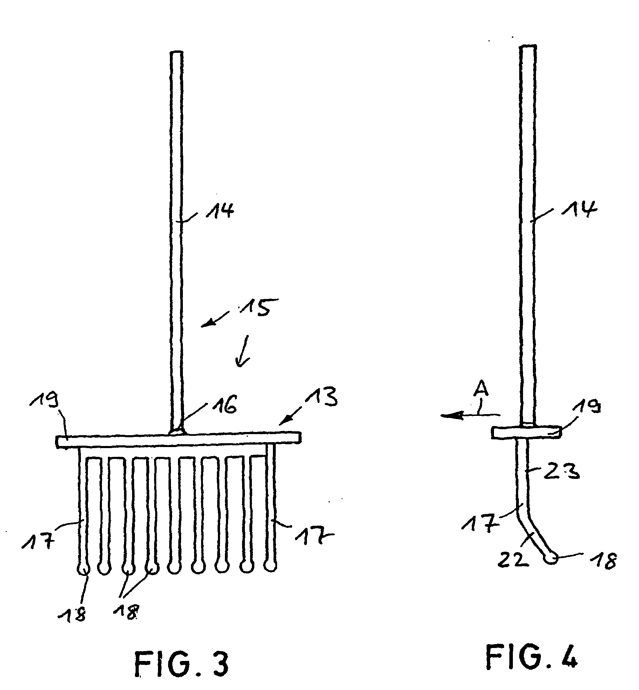

Die Länge der einzelnen Zinken 17 einschließlich der Verdickung 18 ist so gemessen, dass die Verdickung 18 gerade den Nährboden 10 berührt. Dann wird der Deckel 5 relativ zur Schale 1 in Richtung des in den Figuren 1, 2 und 4 gezeigten Pfeiles A gedreht, wobei sich die Zinken 17 im Bereich der Verdickungen 18 entlang der Kreisbahnen 21 über die Nährbodenoberfläche ziehen und hierbei das zu untersuchende Gut, respektive die vom Gut aufgenommenen Keime, entlang der Kreisbahnen 21 auf dem Nährboden 10 verteilen. Wesentlich ist hierbei die gebogene Ausbildung der Zinken 17, wie sie in Figur 4 gezeigt ist und die hierauf bezogene Bewegungsrichtung A des Rechens 13. Durch die Biegsamkeit der Zinken 17, insbesondere des unteren abgebogenen Abschnittes 22 zum oberen senkrecht zur Grundplatte 19 orientierten Abschnitt 23 ist gewährleistet, dass sich die Verdickungen 18 nicht tief in den Nährboden 10 eingraben, sondern über diesen geführt werden.

Gemäß der Vorrichtung kann das zu untersuchende Gut, respektive die Keime, auf einfache Art und Weise in einer Vielzahl von Kreisbahnen 21, entsprechend der Anzahl der Zinken 17, definiert auf der Oberfläche des Nährbodens 10 verteilt werden. Durch den dichten Abschluß des Deckels 5 zur Schale 1 bzw. der Grundplatte 19 des Rechens 13 zum Deckel 5 ist ein geschlossenes System gebildet, d.h. es erfolgt die Keimbildung ausschließlich innerhalb dieses Systems.

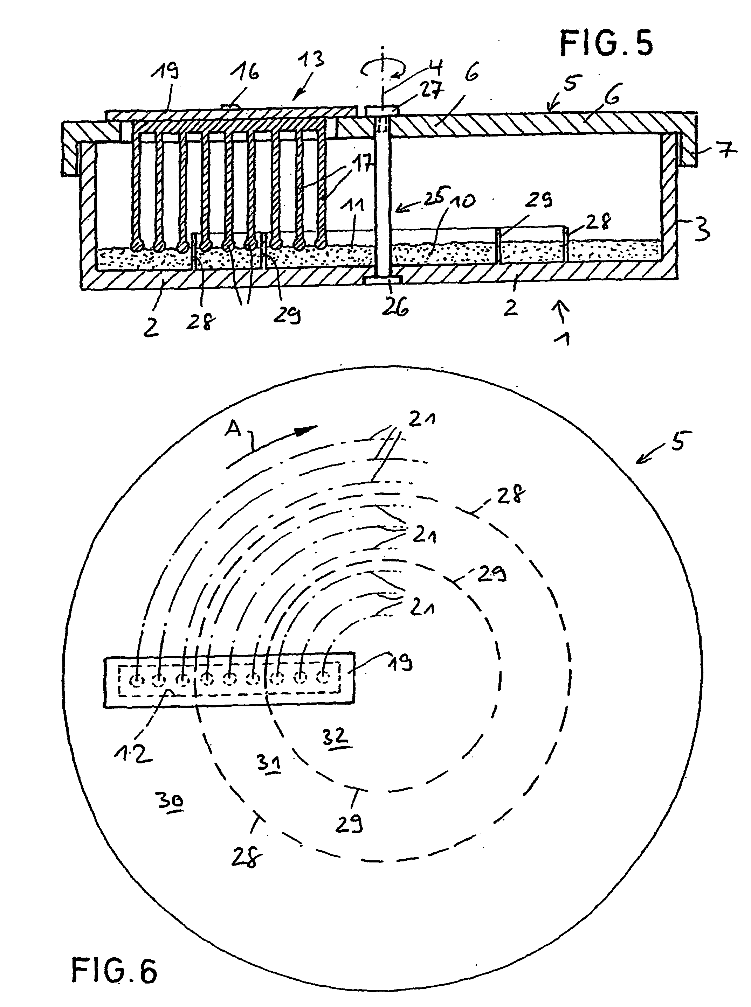

Die Ausführungsform der erfindungsgemäßen Vorrichtung gemäß der Darstellung der Figuren 5 und 6 ist grundsätzlich wie diejenige nach den Figuren 1 bis 4 gestaltet. Die in ihrem Aufbau und ihrer Funktionsweise übereinstimmenden Bauteile sind deshalb der Einfachheit halber mit denselben Bezugsziffern bezeichnet. Bei der Ausführungsform nach den Figuren 5 und 6 erfolgt die Verbindung von Deckel 5 und Schale 1 nicht über den Formschluß von Ringnut 9 und Ringvorsprung 8, sondern mittels einer im Bereich der zentralen Achse 4 angeordneten Steckverbindung 24. Diese weist einen durch ein Loch im Schalenboden 2 gesteckten Stift 25 mit einem einen Rücksprung im Schalenboden 2 kontaktierenden Kopf 26 auf. Der Stift 25 durchsetzt eine Öffnung im Deckel 5, und es ist mit dem freien Ende des Stiftes 25 ein Stopfen 27 verrastet, womit eine feste Verbindung von Stopfen 27 und Stift 25 erfolgt, die den Deckel 5 auf die Schale 1 drückt. Ein weiterer Unterschied ist darin zu sehen, dass bei der Ausführungsform nach den Figuren 5 und 6 im Schalenboden 2 durch zwei konzentrisch zur Achse 4 angeordnete Ringstege 28 und 29 drei voneinander getrennte Nährbodenbereiche 30, 31 und 32 gebildet sind, denen, im Ausführungsbeispiel, jeweils drei Zinken 17 zugeordnet sind. Die Ringstege 28, 29 weisen eine Höhe auf, die größer ist als die Stärke des Nährbodens 10

Vorzugsweise ist die Grundplatte so gestaltet, dass nur ein gerichtetes, somit eindeutiges Einsetzen der Grundplatte und damit der Zinken 17 möglich ist. Dies berücksichtigt die abgewinkelte Ausbildung der Zinken und verhindert, dass der Rechen mit den Zinken in Drehrichtung des Deckels vorlaufend, bezogen auf den Abschnitt 22, eingesetzt wird. Zudem kann ein Anschlag zwischen der Schale und dem Deckel vorgesehen sein, der ein Drehen des Deckels relativ zur Schale von maximal etwa 360° gestattet.

## Patentansprüche

1. Vorrichtung zum Züchten von Keimkulturen, mit einer Schale (1) und einem relativ zu dieser drehbaren Deckel (5), die beim Züchten der Keimkulturen ein geschlossenes System bilden, wobei auf den Boden (2) der Schale (1) ein Nährboden (10) für die Keime aufgebracht ist und Mittel (13) zum Beimpfen des Nährbodens (10) mit Keimen vorgesehen sind, **dadurch gekennzeichnet, dass** der Deckel (5) mit einer Öffnung (12) zur Aufnahme des Mittels (13) zum Beimpfen des Nährbodens (10) versehen ist, wobei das Mittel zum Beimpfen als Rechen (13), Kamm oder Bürste ausgebildet ist, das in den Deckel (5) eingesetzt wird, wobei die Zinken (17) des Rechens (13) bzw. Kamms oder Borsten der Bürste bei mit der Schale (1) verbundenem Deckel (5) und in den Deckel (5) eingesetzter Stellung des Rechens (13), des Kamms oder der Bürste den Nährboden (10) kontaktieren.

2. Vorrichtung nach Anspruch 1, **dadurch gekennzeichnet, dass** der Rechen (13), der Kamm oder die Bürste bei mit dem Deckel (5) versehener Schale (1) von außen in den Deckel (5) eingesetzt wird.

3. Vorrichtung nach Anspruch 1 oder 2, **dadurch gekennzeichnet, dass** die Öffnung (12) des Deckels (5), vor dem Einsetzen des Rechens (13), des Kamms bzw. der Bürste in den Deckel (5), verschlossen ist, insbesondere mittels einer außen auf den Deckel (5) aufgebrachten Klebefolie.

4. Vorrichtung nach einem der Ansprüche 1 bis 3, **dadurch gekennzeichnet, dass** der Rechen (13), der Kamm bzw. die Bürste eine Grundplatte (19) und mit dieser verbundene Zinken (17) bzw. Borsten aufweist, wobei die Abmessungen von Grundplatte (19) und Öffnung (12) im Deckel (5) so bemessen sind, dass bei in den Deckel (5) eingesetztem Rechen (13), Kamm bzw. Bürste die Grundplatte (19) den die Deckelöffnung (12) begrenzenden Deckelrand abdeckt.

5. Vorrichtung nach Anspruch 4, **dadurch gekennzeichnet, dass** die Grundplatte (19) im Überlappungsbereich mit dem Deckel (5) mit dem Deckel (5) verbunden ist, insbesondere mittels einer Klebverbindung.

6. Vorrichtung nach einem der Ansprüche 1 bis 5, **dadurch gekennzeichnet, dass** der Querschnitt der Öffnung 812) im Deckel (5) und die auf die Einsteckrichtung des Rechens (13), des Kamms bzw. der Bürste bezogene projizierte Fläche der Grundplatte (19) rechteckig ist, wobei die projizierte Fläche der Grundplatte (19) größer ist als der Querschnitt der Öffnung (12) im Deckel (5).

7. Vorrichtung nach einem der Ansprüche 1 bis 6, **dadurch gekennzeichnet, dass** die Schale (1) und der Deckel (5) rotationssymmetrisch ausgebildet sind.

8. Vorrichtung nach Anspruch 7, **dadurch gekennzeichnet, dass** der Rechen (13), der Kamm bzw. die Bürste radial ausgerichtet ist.

9. Vorrichtung nach einem der Ansprüche 1 bis 8, **dadurch gekennzeichnet, dass** das Ende (18) der jeweiligen Zinke (19) des Rechens (13) bzw. Kamms als Kugel, Halbkugel, Spitze oder Impföse ausgebildet ist.

10. Vorrichtung nach einem der Ansprüche 1 bis 9, **dadurch gekennzeichnet, dass** die jeweilige Zinke (17) im Bereich ihres freien Endes (18), insbesondere über bis zu ½ ihrer Länge, in einem stumpfen Winkel abgewinkelt ist, derart, dass das freie Ende, bezogen auf die Drehrichtung (A) des Deckels (5) zur Schale (1), mit dem den Zinken (17) vorlaufend angeordneten Bereich des Schalenbodens (2) einen spitzen Winkel einschließt.

11. Vorrichtung nach einem der Ansprüche 1 bis 10, **dadurch gekennzeichnet, dass** mit der Grundplatte (19), auf ihrer den Zinken (17) bzw. Borsten abgewandten Seite, ein Stiel (14) verbunden ist.

12. Vorrichtung nach Anspruch 11, **dadurch gekennzeichnet, dass** der Verbindungsbereich von Stiel (14) und Grundplatte (19) als Sollbruchstelle (16) ausgebildet ist.

13. Vorrichtung nach einem der Ansprüche 1 bis 12, **dadurch gekennzeichnet, dass** die Schale (1) durch ein oder mehrere, im Bereich des Bodens (2) angeordnete, konzentrisch zur Rotationsachse (4) von Schale (2) und Deckel (5) verlaufende Stege (28, 29) in Nährbodenbereiche (32, 31, 30) unterteilt ist.

14. Vorrichtung nach einem der Ansprüche 1 bis 13, **dadurch gekennzeichnet, dass** die Schale (1) und der Deckel (5) mittels Rastmitteln (7, 8) miteinander verrastbar oder mittels eines separaten Halteelementes (25, 27) miteinander verbindbar sind.

15. Vorrichtung nach einem der Ansprüche 1 bis 14, **dadurch gekennzeichnet, dass** der Deckel (5) mit einer weiteren Öffnung versehen ist, die ein Verschlußelement, insbesondere einen Gummistopfen durchsetzt.

## Claims

1. Device for growing germ cultures, with a dish (1) and a lid (5) rotatable with respect thereto which form a closed system for growing germ cultures, wherein a nutrient medium (10) for the germs is put on the bottom (2) of the dish (1) and means (13) for inoculating the nutrient medium (10) with germs are provided, **characterized in that** the lid (5) is provided with an opening (12) to receive the means (13) for inoculating the nutrient medium (10), the said inoculation means being configured as a rake (13), comb or brush which is inserted into the lid (5), the prongs (17) of the rake (13), teeth of the comb or bristles of the brush contacting the nutrient medium (10) when the lid (5) is fitted to the dish (1) and the rake (13), comb or brush is in its inserted position in the lid (5).

2. Device according to Claim 1, **characterized in that** the rake (13), comb or brush is inserted into the lid (5) from outside after the lid (5) has been fitted to the dish (1).

3. Device according to Claim 1 or Claim 2, **characterized in that** before the rake (13), comb or brush is inserted into the lid (5), the opening (12) in the lid (5) is closed, in particular by means of an adhesive foil placed on the outside of the lid (5).

4. Device according to any one of Claims 1 to 3, **characterized in that** the rake (13), comb or brush has a base plate (19) and prongs (17), teeth or bristles, respectively, attached thereto, the dimensions of the base plate (19) and the opening (12) in the lid (5) being chosen so that the rim of the lid opening (12) is covered by the base plate (19) when the rake (13), comb or brush is inserted in the lid (5).

5. Device according to Claim 4, **characterized in that** where the base plate (19) overlaps the lid (5) it is attached to the lid (5), in particular by an adhesive-bonded joint.

6. Device according to any one of Claims 1 to 5, **characterized in that** the cross-section of the opening (12) in the lid (5) and the projected area of the base plate (19) in the direction of insertion of the rake (13), comb or brush [are both] rectangular, the projected area of the base plate (19) being greater than the cross-section of the opening (12) in the lid (5).

7. Device according to any one of Claims 1 to 6, **characterized in that** the dish (1) and lid (5) are rotationally symmetrical.

8. Device according to Claim 7, **characterized in that** the rake (13), comb or brush is radially orientated.

9. Device according to any one of Claims 1 to 8, **characterized in that** the tip (18) of each prong (19)[*sic*] of the rake (13), or of each tooth of the comb, is configured as a sphere, hemisphere, spike, or inoculation needle.

10. Device according to any one of Claims 1 to 9, **characterized in that** each prong (17) or tooth is bent at an obtuse angle in the region of its free end (18), in particular over up to half its length, so that the tip includes an acute angle with the region of the dish bottom (2) ahead of the prongs (17) or teeth, that is to say, "ahead" in the direction of rotation (A) of the lid (5) with respect to the dish (1).

11. Device according to any one of Claims 1 to 10, **characterized in that** a handle (14) is joined to the base plate (19) on the opposite side to the prongs (17), teeth or bristles.

12. Device according to Claim 11, **characterized in that** the joint between handle (14) and base plate (19) is configured as a predetermined breaking point (16).

13. Device according to any one of Claims 1 to 12, **characterized in that** the dish (1) is divided into culture-medium zones (32, 31, 30) by one or more dividing webs (28, 29) arranged in the region of the bottom (2) and extending concentrically around the rotational axis (4) of dish (2)[*sic*] and lid (5).

14. Device according to any one of Claims 1 to 13, **characterized in that** the the dish (1) and lid (5) are fastenable to each other by snap-fastening means (7, 8) or attachable to each other by means of a separate retaining element (25, 27).

15. Device according to any one of Claims 1 to 14, **characterized in that** the lid (5) is provided with a further opening through which a stopper element, in particular a rubber plug, is inserted.

## Revendications

1. Dispositif pour la croissance de culture de germes, comprenant une capsule (1) et un couvercle (5) rotatif par rapport à cette dernière, qui forment un système fermé lors de la croissance des germes, un milieu de culture (10) pour les germes étant appliqué sur le fond (2) de la capsule (1) et des moyens (13) étant prévus pour l'ensemencement du milieu de culture (10) avec des germes, **caractérisé en ce que** le couvercle (5) est muni d'une ouverture (12) pour la réception du moyen (13) pour l'ensemencement du milieu de culture (10), le moyen pour l'ensemencement étant réalisé sous la forme d'un râteau (13), d'un peigne ou d'une brosse que l'on insère dans le couvercle (5), les griffes (17) du râteau (13), respectivement du peigne ou les poils de brosserie de la brosse, dans l'état dans lequel le couvercle (5) est relié à la capsule (1) et dans la position dans laquelle le râteau (13), le peigne ou la brosse est inséré dans le couvercle (5), entrant en contact avec le milieu de culture (10).

2. Dispositif selon la revendication 1, **caractérisé en ce que** le râteau (13), le peigne ou la brosse, lorsque la capsule (1) est munie du couvercle (5), est inséré dans le couvercle (5) depuis l'extérieur.

3. Dispositif selon la revendication 1 ou 2, **caractérisé en ce que** l'ouverture (12) du couvercle (5), avant l'insertion du râteau (13), du peigne, respectivement de la brosse dans le couvercle (5), est fermée, en particulier à l'aide d'une feuille adhésive appliquée à l'extérieur du couvercle (5).

4. Dispositif selon l'une quelconque des revendications 1 à 3, **caractérisé en ce que** le râteau (13), le peigne, respectivement la brosse présente une plaque de base (19) et des griffes (17), respectivement des poils de brosserie reliés à ladite plaque, les dimensions de la plaque de base (19) et de l'ouverture (12) dans le couvercle (5) étant calculées de telle sorte que, dans l'état dans lequel le râteau (13), le peigne, respectivement la brosse est inséré dans le couvercle (5), la plaque de base (19) recouvre le bord du couvercle délimitant l'ouverture (12) du couvercle.

5. Dispositif selon la revendication 4, **caractérisé en ce que** la plaque de base (19) est reliée au couvercle (5) dans la zone de chevauchement avec le couvercle (5).

6. Dispositif selon l'une quelconque des revendications 1 à 5, **caractérisé en ce que** la section transversale de l'ouverture (12) dans le couvercle (5) et la surface de projection de la plaque de base (19) par rapport à la direction d'enfichage du râteau (13), du peigne, respectivement de la brosse, sont rectangulaires, la surface de projection de la plaque de base (19) étant supérieure à la section transversale de l'ouverture (12) dans le couvercle (5).

7. Dispositif selon l'une quelconque des revendications 1 à 6, **caractérisé en ce que** la capsule (1) et le couvercle (5) sont réalisés de manière symétrique en rotation.

8. Dispositif selon la revendication 7, **caractérisé en ce que** le râteau (13), le peigne, respectivement la brosse est orienté en direction radiale.

9. Dispositif selon l'une quelconque des revendications 1 à 8, **caractérisé en ce que** l'extrémité (18) de la griffe respective (19) du râteau (13), respectivement du peigne est réalisée sous la forme d'une boule, d'une demi-boule, d'une pointe ou d'une anse d'ensemencement.

10. Dispositif selon l'une quelconque des revendications 1 à 9, **caractérisé en ce que** la griffe respective (17), dans la zone de son extrémité libre (18), en particulier sur une distance s'étendant jusqu'à la moitié de sa longueur, est coudée en formant un angle obtus, de telle sorte que, l'extrémité libre, rapporté à la direction de rotation (A) du couvercle (5) par rapport à la capsule (1), forme un angle aigu avec la zone de la base (2) de la capsule disposée à l'avant des griffes (17).

11. Dispositif selon l'une quelconque des revendications 1 à 10, **caractérisé en ce qu'**une tige (14) est reliée à la plaque de base (19) sur le côté de cette dernière se détournant des griffes (17), respectivement des poils de brosserie.

12. Dispositif selon la revendication 11, **caractérisé en ce que** la zone de liaison de la tige (14) et de la plaque de base (19) est réalisée sous la forme d'un point (16) destiné à la rupture.

13. Dispositif selon l'une quelconque des revendications 1 à 12, **caractérisé en ce que** la capsule (1) est subdivisée en zones de milieu de culture (32, 31, 30) par une ou plusieurs nervures (28, 29) disposées dans la zone de la base (2) et s'étendant en position concentrique par rapport à l'axe de rotation (4) de la capsule (1) et du couvercle (5).

14. Dispositif selon l'une quelconque des revendications 1 à 13, **caractérisé en ce que** la capsule (1) et le couvercle (5) peuvent être reliés l'un à l'autre à l'aide de moyens d'encliquetage (7, 8) ou peuvent être reliés l'un à l'autre à l'aide d'un élément d'arrêt séparé (25, 27).

15. Dispositif selon l'une quelconque des revendications 1 à 14, **caractérisé en ce que** le couvercle (5) est muni d'une ouverture supplémentaire qui traverse un élément de fermeture, en particulier un bouchon en caoutchouc.
